# EUROPEAN PATENT SPECIFICATION

(11) **EP 0 369 671 B1**
(45) Date of publication and mention of the grant of the patent: **20.04.1994**
(21) Application number: 89311561.8
(22) Date of filing: 08.11.1989
(51) Int. Cl.: G07C 9/00, A61B 5/117

(54) **Personal identification arrangements**
Personalidentifizierungsanordnungen
Dispositif d'identification personnelle

(30) Priority: 15.11.1988 GB 8826696
(43) Date of publication of application: 23.05.1990
(73) Proprietor: THORN EMI plc, London W1A 2AY (GB)
(72) Inventor: Cattell, Alan Frank, Holner Green Buckinghamshire HP15 6TQ (GB); Walters, Peter Ernest, Hanwell London W7 3BX (GB)
(74) Representative: Hurst, Richard Arthur Alexander

(56) References cited:
- EP-A- 0 045 915
- EP-A- 0 244 498
- US-A- 3 944 978
- US-A- 4 322 163
- US-A- 4 455 083
- US-A- 4 641 350

## Description

This invention relates to personal identification arrangements and it relates more particularly to such arrangements as operate by measurement of anatomical characteristics. The arrangements may find application in any area where strict personal identification is necessary, for example in the identification of personnel who are allowed access to secure premises or in the identification of an unauthorized signatory allowed to sanction the electronic transfer of funds or for forensic purposes.

It is generally accepted that personal identification arrangements based on anatomical characteristics have significant potential advantages over arrangements which do not use such characteristics, such as card-based systems with or without personal identification numbers (PIN'S). For example, a anatomical characteristic cannot be mislaid (though it is accepted that some characteristics might be altered) and cannot be delegated by loan, for example to unauthorised persons, or otherwise compromised, as in the case where PIN's are written down or set to obvious values.

Difficulties arise, however, in implementing arrangements based on the measurement of anatomical characteristics because of the complexity and expense involved in capturing data indicative of the characteristic in question, in processing that data to derive therefrom recognition criteria having a suitable degree of security and in storing such criteria and information relating thereto.

EP-A-0,244,498 discloses an arrangement which uses a portable data carrier in the form of a plastic card which has several magnetic tracks for receiving data. A fingerprint recording terminal with a sensor contains a memory, in which reference signal images are stored for comparison with a currently-analysed fingerprint. The degree of correlation is compared with the data on the card.

US-A-3,944,978 discloses a system wherein coherent light is projected through a prism to its hypotenuse, contacting which is a fingerprint to be identified. The diffraction pattern generated is then frequency-domain analysed to provide an electrical function which is used for comparison with other such functions.

The present invention aims to reduce at least the difficulty associated with capturing data indicative of the characteristics in question; typical characteristics for which use of the invention is appropriate including fingerprints and palm prints and, in general any characteristic print which can be captured upon contact of a body portion with a suitably responsive medium, or with a surface with which said responsive medium can subsequently be brought into contact.

A personal identification arrangement including:
a receiver for receiving a representation of an anatomical characteristic of a person to be identified, the receiver characterised by including means for establishing contact between a metallic thin film and the representation to allow the representation to cause changes in surface plasmon resonance;
a signal generator to respond to such changes in surface plasmon resonance by providing electrical signals indicative of these changes;
and a signal processor to process the electrical signals to generate recognition data representative of the changes and hence the characteristic, for comparison with stored recognition data.

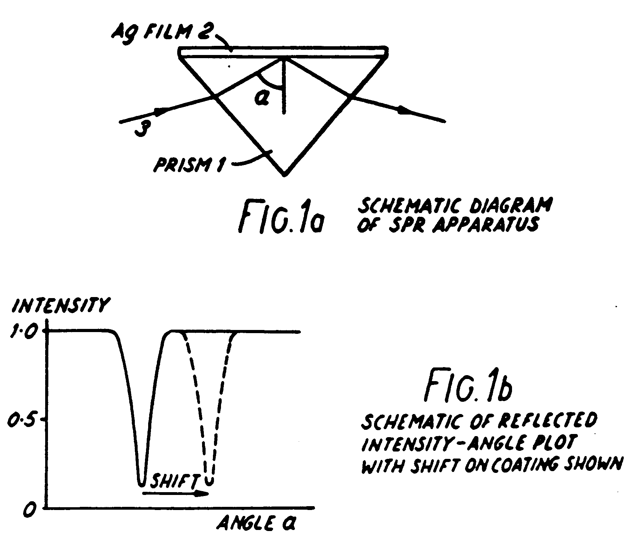

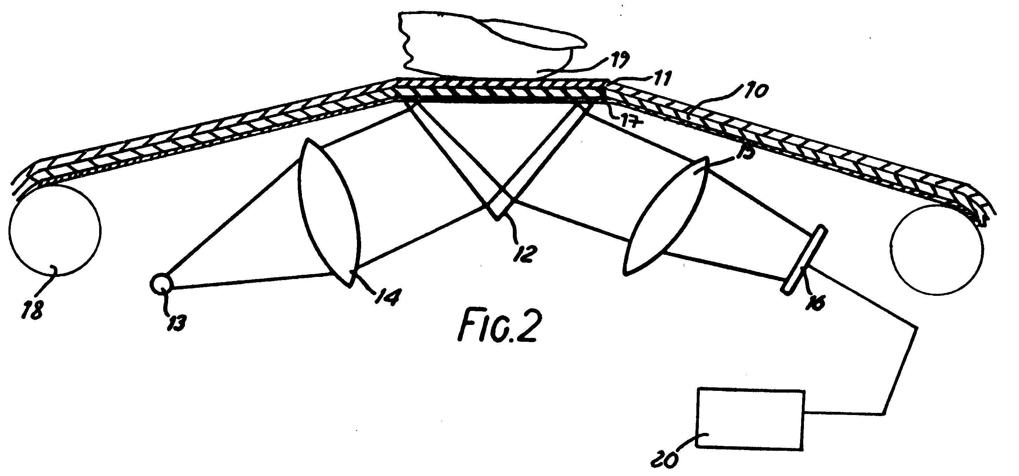

In order that the invention may be clearly understood and readily carried into effect, the phenomenon of surface plasmon resonance (SPR) will now be described with reference to Figures 1(a) and 1(b) of the accompanying drawings and an embodiment of the invention will be described with reference to Figure 2 of the accompanying drawings.

In the drawings,
Figures 1(a) and 1(b) are schematic diagrams used to support an explanation of the phenomenon of SPR, and Figure 2 shows a schematic layout of an arrangement in accordance with one example of the invention.

Referring now to Figures 1(a) and 1(b), the phenomenon of SPR can be described as follows:-
Onto one surface of a prism 1 is coated a thin film 2 of silver of thickness approximately 50nm. Collimated light 3 is caused to impinge onto the silvered surface as shown in Figure 1(a) and the angle of incidence (a) is varied. The intensity of the reflected light 4 will vary with angle of incidence as shown in Figure 1(b). The sharp dip in the reflected intensity is due to SPR. At this angle the incident light 3 is coupled to plasmon modes in the outer surface of the silver film 2. The angle at which this coupling occurs is very sensitive to the refractive index of the medium at the surface of the silver film, and changes of 1 part in 10⁵ in this refractive index can be measured relatively easily.

Figure 1(b) shows schematically the change in the reflectance characteristics that would occur if the silver were to be coated with 10nm of a typical organic film. It can be seen that the resonance typically shifts by more than its width. It is therefore clear that this phenomenon can be used to detect the presence of very thin layers on the surface of the metal layer.

The sensitivity of SPR to layers on the surface of a thin film of silver can be exploited for finger print reading as follows. When a finger is brought into contact with the silver, a quantity of material will be deposited onto the film in a characteristic finger print pattern. This surface is scanned using an incident beam at the angle corresponding to the resonance minimum of a clear silver film, so that only in the regions where there is a shift in the resonance angle will a significant reflected signal be detected. Since a layer of thickness 10 nm will shift the resonance by more that the resonance width, there will be a substantial contrast between those regions where the print pattern exists and where it does not. Of course, the use of silver coated prisms for this work is not necessarily desirable. It is however possible to use metallised tape, as follows:

Referring now to Figure 2, a polymer tape 10 with refractive index similar to that of a glass prism, (e.g. cellulose acetate) is coated with a thin silver layer 11. Then, by bringing this tape into intimate contact with the top surface of a prism, a resonance can be observed in exactly the same manner as before. It is however important to index match the tape to the prism using a matching medium 17 but this can conveniently be an elastomeric material, integral with the tape. The advantages of using the tape are considerable. Prints can be taken remotely and subsequently read into the instrument. Moreover, the use of a reel of tape 18 allows the system to be installed in a door as part of an access control system. Each attempt to gain access requires the finger print 19 to be read using a new portion of the tape. A permanent record of access and/or attempted access can be maintained in this way.

Some of the advantages of this arrangement for print reading are that the reader is of relatively low cost, the tape system ensures a clean surface for every print, the contrast of the image is high and a permanent record of those using the access system is maintained on the used silver tape. The main disadvantage is that the tape has to be replaceable or some cleaning system has to be installed with the access system.

Referring again to Figure 2, the tape 10, with its thin silver layer 11 is coupled to a prism 12 which forms part of an SPR reading device. Input light from a source 13 is collimated by an input lens 14 and caused to impinge on that surface of the prism bearing the tape 10 at the angle appropriate for SPR to occur in a clean silver layer. Any light emerging from the output face of prism 12 results from the presence of components of the captured fingerprint representation on the silver layer 11 and this light is focused by means such as a hemispherical lens 15 onto an array 16 of light sensitive detectors. Typically the array comprises a pillar array containing 256 detectors, thus capable of resolving the output light to 256 elements disposed across the film. Lens 15 may comprise a lens, or assembly of lenses, of configuration more complex than hemispherical if desired.

Other arrangements, including 2-dimensional arrays of detectors and/or physical scanning arrangements can be used if desired, even though these are not shown in detail in the two-dimensional schematic of Figure 2. These arrays of light sensitive detectors may be employed to produce electrical signals indicative of the captured representation of the physical characteristic which are then displayed in the form of an X-Y display.

Alternatively, CCD arrays may be used and the acquired information simply stored into RAM 20 such that the instantaneous readings may be compared to previously stored characteristics.

Moreover, it will be appreciated that an impression of some physical characteristic may be captured on some other surface than that of the tape 10, for example by a residue of perspiration, grease etc. being left on that other surface following contact thereof by a body portion. In that case, the tape 10 may subsequently be caused to assume intimate contact with said other surface with the object of picking up the, or some of the, aforesaid residue therefrom.

## Claims

1. A personal identification arrangement including:
a receiver (10, 12) for receiving a representation (19) of an anatomical characteristic of a person to be identified, the receiver (10,12) characterised by including means for establishing contact between a metallic thin film (11) and the representation (19) to allow the representation (19) to cause changes in surface plasmon resonance;
a signal generator (12, 15, 16) to respond to such changes in surface plasmon resonance by providing electrical signals indicative of these changes;
and a signal processor (20) to process the electrical signals to generate recognition data representative of the changes and hence the characteristic, for comparison with stored recognition data.

2. An arrangement according to Claim 1 wherein the metallic thin film (11) is made substantially of silver.

3. An arrangement according to either Claim 1 or Claim 2 wherein the receiver comprises a tape (10) coated with the metallic thin film (11).

4. An arrangement according to any one of the preceding claims wherein the signal generator (12, 15, 16) comprises a prism (12), focussing means (15) and light sensitive detectors (16).

5. An arrangement according to Claim 4 wherein the fosussing means (15) comprises at least one lens.

6. An arrangement according to any one of Claims 1-3 wherein the signal generator (12, 15, 16) includes an array of light sensitive detectors.

7. An arrangement according to any one of Claims 1-3 wherein the signal generator (12, 15, 16) includes a 2-dimensional array of light sensitive detectors.

8. An arrangement according to any one of Claims 1-3 wherein the signal generator (12, 15, 16) includes a physical scanning arrangement.

9. An access control system comprising a personal identification arrangement according to any one of the preceding claims.

## Patentansprüche

1. Personen-Identifizierungsanordnung mit:
einem Empfänger (10, 12) zum Empfang einer Darstellung (19) einer anatomischen Eigenschaft einer zu identifizierenden Person, wobei der Empfänger (10, 12) dadurch gekennzeichnet ist, daß er Mittel zur Herstellung eines Kontakts zwischen einem metallischen Dünnfilm (11) und der Darstellung (19) enthält, um zuzulassen, daß die Darstellung (19) Änderungen in der Oberflächen-Plasmon-Resonanz bewirkt;
einem Signalgenerator (12, 15, 16), der auf derartige Änderungen in der Oberflächen-Plasmon-Resonanz anspricht, indem er elektrische Signale erzeugt, die ein Maß dieser Änderungen sind;
einem Signalprozessor (20) für die Verarbeitung der elektrischen Signale, um Erkennungsdaten zu erzeugen, die die Änderungen und damit die Eigenschaft für einen Vergleich mit gespeicherten Erkennungsdaten darstellen.

2. Anordnung nach Anspruch 1, bei der der metallische Dünnfilm (11) im wesentlichen aus Silber hergestellt ist.

3. Anordnung nach Anspruch 1 oder 2, bei der der Empfänger ein mit dem metallischen Dünnfilm beschichtetes Band (10) umfaßt.

4. Anordnung nach einem der vorhergehenden Ansprüche, bei der der Signalgenerator (12, 15, 16) ein Prisma (12), Fokussiermittel (15) und lichtempfindliche Detektoren (16) umfaßt.

5. Anordnung nach Anspruch 4, bei der die Fokussierungsmittel (15) wenigstens eine Linse enthalten.

6. Anordnung nach einem der Ansprüche 1 bis 3, bei der der Signalgenerator (12, 15, 16) eine Gruppe von lichtempfindlichen Detektoren enthält.

7. Anordnung nach einem der Ansprüche 1 bis 3, bei der der Signalgenetator (12, 15, 16) eine 2-dimensionale Gruppe von lichtempfindlichen Detektoren enthält.

8. Anordnung nach einem der Ansprüche 1 bis 3, bei der der Signalgenerator (12, 15, 16) eine physikalische Abtastanordnung enthält.

9. Zugangs-Kontrollsystem mit einer Personen-Identifizierungsanordnung nach einem der vorhergehenden Ansprüche.

## Revendications

1. Dispositif d'identification de personne comprenant:
un récepteur(10,12) pour recevoir une représentation (19) d'une caractéristique anatomique d'une personne à identifier, le récepteur (10,12) étant caractérisé en ce qu'il inclut des moyens pour établir un contact entre un mince film métallique (11) et la représentation (19) afin de permettre à la représentation (19) d'induire des modifications dans une résonance plasmon de surface;
un générateur de signal (12, 15, 16) pour répondre à de telles modifications dans la résonance plasmon de surface en fournissant des signaux électriques indiquant ces modifications; et
un processeur de signal (20) pour traiter les signaux électriques afin de produire des données de reconnaissance représentatives des modifications et donc de la caractéristique.

2. Dispositif conforme à la revendication 1 dans lequel le mince film métallique (11) est réalisé principalement en argent.

3. Dispositif conforme à la revendication 1 ou 2 dans lequel le récepteur comprend un ruban (10) qui est revêtu d'un mince film métallique (11).

4. Dispositif conforme à l'une quelconque des revendications précédentes dans lequel le générateur de signal (12, 15, 16) comprend un prisme (12), des moyens de focalisation (15) et des détecteurs sensibles à la lumière (16).

5. Dispositif conforme à la revendication 4 dans lequel les moyens de focalisation (15) comprennent au moins une lentille.

6. Dispositif conforme à l'une quelconque des revendications 1 à 3 dans lequel le générateur de signal (12, 15, 16) inclut un réseau de détecteurs sensibles à la lumière.

7. Dispositif conforme à l'une quelconque des revendications 1 à 3 dans lequel le générateur de signal (12, 15, 16) inclut un réseau à deux dimensions de détecteurs sensibles à la lumière.

8. Dispositif conforme à l'une quelconque des revendications 1 à 3 dans lequel le générateur de signal (12, 15, 16) inclut un dispositif de balayage spatial.

9. Système de contrôle d'accès comprenant un dispositif d'identification de personne conforme à l'une quelconque des revendications précédentes.
